(19) Europäisches Patentamt
European Patent Office
Office européen des brevets

(11) Publication number: **0 252 028 A1**

(12) **EUROPEAN PATENT APPLICATION**

(21) Application number: 87830236.3

(22) Date of filing: 24.06.87

(51) Int. Cl.³: C 07 D 405/04
A 61 K 31/415, A 61 K 31/34-5

(30) Priority: 25.06.86 IT 4351486

(43) Date of publication of application: 07.01.88 Bulletin 88/1

(84) Designated Contracting States: AT BE CH DE ES FR GB GR LI LU NL SE

(71) Applicant: Sigma-Tau Industrie Farmaceutiche Riunite S.p.A.
47, Viale Shakespeare
I-00144 Rome(IT)

(72) Inventor: Spazzoni, Paola
12, Via XX Settembre
Marsciano I-06100 Perugia(IT)

(74) Representative: Fassi, Aldo, Dr.
c/o Sigma-Tau Industrie Farmaceutiche Riunite S.p.A. Via Pontina, Km. 30, 400
I-00040 Pomezia (Roma)(IT)

(54) **Process for preparing nitrofurylbenzimidazoles and pharmaceutical compositions having antimycotic, antibacterial and antitubercular activities containing same.**

(57) Nitrofurylbenzimidazoles having general formula (I)

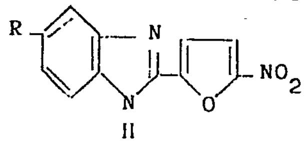

wherein R is
hydrogen;
straight or branched $C_1-C_{12}$ alkyl;
halogen;
nitro group; or
halogen-substituted $C_1-C_4$ lower alkyl are obtained with high yields by condensing an o-phenylendiamine having general formula (II)

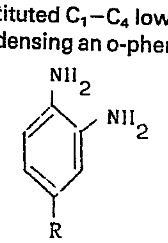

wherein R has the above-identified meaning, with 5-nitro-2-furaldehyde in the presence of a ferricyanide of the alkaline or alkaline-earth metals.

Nitrofurylbenzimidazoles (I) are endowed with potent antimycotic, antibacterial and antitubercular activities and are suitable for being compounded into pharmaceutical compositions administrable via the systemic route.

EP 0 252 028 A1

Process for preparing nitrofurylbenzimidazoles and pharmaceutical compositions having antimycotic, antibacterial and antitubercular activities containing same.

The present invention relates to a process for preparing nitrofurylbenzimidazoles and to pharmaceutical compositions containing same, which are administrable via the systemic route, having antimycotic, antibacterial and antitubercular activities.

Although the process of the present invention is suitable for preparing variably substituted nitrofurylbenzimidazoles regardless of nature of substituents, it has been found that this process is particularly advantageous for preparing nitrofurylbenzimidazoles having general formula (I)

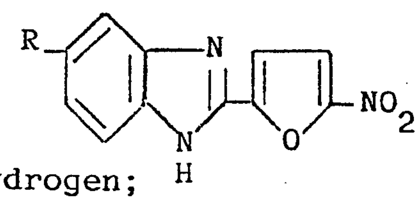

(I)

wherein R is hydrogen;

straight or branched $C_1$-$C_{12}$ alkyl;

halogen;

nitro group; or

halogen-substituted $C_1$-$C_4$ lower alkyl.

Preferably, the halogen is fluorine and the halogen-substituted lower alkyl is trifluoromethyl.

The compounds of general formula (I) are known already: see e.g. UK patents 1,072,735 and 1,094,903 which, inter alia, disclose the antibacterial and anthelminthic activities of these compounds. Moreover, Alunni Bistocchi G. et al. in Il Farmaco - Ed. Sc.Vol.37 fasc.9, 597-611 (1982) and vol. 39, fasc.8, 660-673 (1984) disclose the potent antibacterial and antimycotic activities of the compound (I) wherein R is fluorine, i.e. of 5-fluoro-2-(5'-nitro-2'-furyl)-benzimidazole.

It is known that benzimidazoles are synthesized by reacting

o-phenylendiamines with aldehydes or organic acids.

Thus, for instance, in order to prepare alkylbenzimidazoles, o-phenylendiamine is reacted with aliphatic acids or aldehydes.

In order to carry out this reaction, oxidizing or cyclizing agents, such as lead tetra-acetate, cupric acetate, boric acid, and the like are required. More specifically, alkylbenzimidazoles have been prepared by reacting o-phenylendiamines with aldehydes, using as oxidizing agent: cupric acetate (R.Weidenhagen, Ber., 69, 2263 (1963)), or lead tetra-acetate (M.Terashima, Synthesis, 1982, 484, (1982)), or by reacting o-phenylendiamines with organic acids using boric acid or ethyl polyphosphate as cyclizing agents (G.L. Dunn et al., J.Med.chem. 9, 751, (1966); G.A.Bistocchi et al., Coll.Czech. Commun., 50,1959, (1985)).

When the known methods for preparing benzimidazoles are used for synthesizing nitrofurylbenzimidazoles, it is found that, in several cases, remarkable difficulties are encountered and the yield is generally very low.

Particularly, in the preparation of 5-fluoro-2-(5'-nitro-2'-furyl)-benzimidazole, a yield of 2% only (calculated on the starting 4-fluoro-o-phenylendiamine) is obtained in the presence of cupric acetate, whereas in the presence of lead tetra-acetate the yield is actually nil.

The process for preparing the nitrofurylbenzimidazoles of general formula (I) in accordance with the present invention comprises condensing an o-phenylendiamine having general formula (II)

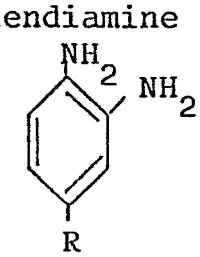

(II)

wherein R is hydrogen,

straight or branched $C_1$-$C_{12}$ alkyl,

halogen;

nitro-group; or

halogen-substituted $C_1$-$C_4$ lower alkyl.

with 5-nitro-2-furaldehyde in the presence of an oxidizing agent and is characterized in that the oxidizing agent is selected from the ferricyanides of the alkaline and alkaline-earth metals.

Preferably, the ferricyanide is selected from the sodium potassium, rubidium, cesium, calcium, stronzium, barium and magnesium ferricyanides.

The condensation reaction is carried out by contacting warm o-phenylendiamine (II) with 5-nitro-2-furaldehyde in aqueous-alcoholic solvents in the presence of the above-identified ferricyanides.

The reaction products are purified via column chromatography and/or crystallization.

In contrast with the prior art methods, the process of the present invention allows the nitrofurylbenzimidazoles (I) to be obtained with remarkably high yields, ranging from about 50 to 85% calculated on o-phenylendiamine.

The process of the present invention entails further advantages over the known methods. For instance, in carrying out the method disclosed in the above-mentioned UK patent 1,094,903 it is mandatory that all traces of water be removed from the reactants and that the reaction mixture be protected from contamination with atmospheric moisture during the whole course of the reaction. Otherwise, in the presence of water rapid hydrolysis of the reactant featuring this synthesis route (i.e. the addition salt of imidic acid) may ensue. This makes the process hardly feasible on an industrial scale. Conversely, the process of the present invention is carried out in an aqueous-alcoholic environment, and particular precautions in its operation need not be taken.

The following non-limiting examples illustrate the preparation of some nitrofurylbenzimidazoles (I) via the process of the present invention.

<u>Example 1:</u> Preparation of 2-(5'-nitro-2'-furyl)-benzimidazole

Into a 1-liter three necked round-bottom flask equipped with stirrer, thermometer, reflux condenser and loading funnel, placed on a thermostatic bath, were introduced:

| | |
|---|---|
| 5-nitro-2-furaldehyde | 15.6 g |
| o-phenylendiamine | 10.0 g |
| methanol | 200.0 ml |

The mixture was kept under stirring until dissolution of the reactants.

To the solution thus obtained, a solution of 67.0 g of potassium ferricyanide in 100 ml of water was added at room temperature.

The reaction mixture was heated up to its boiling point and the reaction was continued at this temperature for two hours.

The product thus obtained was filtered by suction through Kieselguhr.

The residue was washed with ethanol.

The filtrate and the washing solvent were combined and concentrated to a small volume by evaporation under vacuum.

Crushed ice was added to precipitate the raw reaction product.

The purification of the raw product was carried out by fractional crystallization from an ethanol/water (80/20 v/v) mixture.

The 2-(5'-nitro-2'-furyl)-benzimidazole thus obtained (yield 54% calculated on o-phenylendiamine) had the following characteristics:

-appearance : yellowish crystalline solid

-melting point: 226-227°C

-elementary analysis (calculated for $C_{11}H_7O_3N_3$):

| | | | |
|---|---|---|---|
| found: | C=57.43% | H=3.11% | N=18.20% |
| calculated: | C=57.64% | H=3.06% | N=18.34% |

Example 2: Preparation of 5-methyl-2-(5'-nitro-2'-furyl)-benzimidazole.

The procedure of Example 1 was followed, preparing a solution of

| | | |
|---|---|---|
| 5-nitro-2-furaldehyde | 13.9 | g |
| 4-methyl-o-phenylendiamine | 10.0 | g |
| methanol | 200.0 | ml |

To this solution, a solution of 59.3 g of potassium ferricyanide in 100 ml of water was added at room temperature.

The reaction mixture was heated up to its boiling point and the reaction was continued at this temperature for three hours.

Filtration, washing, concentration, precipitation and purification as in Example 1.

The 5-methyl-2-(5'-nitro-2'-furyl)-benzimidazole thus obtained (yield 51% calculated on 4-methyl-o-phenylendiamine) had the following characteristics:

-appearance : yellow crystalline solid

-melting point : 222-223°C

-elementary analysis (calculated for $C_{12}H_9O_3N_3$):

| | | | |
|---|---|---|---|
| found: | C=59.05% | H=3.78% | N=17.17% |
| calculated: | C=59.26% | H=3.70% | N=17.28% |

0252028

Example 3: Preparation of 5-fluoro-2-(5'-nitro-2'-furyl)-benzimidazole.

The procedure of Example 1 was followed, preparing a solution of

| | | |
|---|---|---|
| 5-nitro-2-furaldehyde | 13.4 | g |
| 4-fluoro-o-phenylendiamine | 10.0 | g |
| methanol | 200.0 | ml |

To this solution, a solution of 57.4 g of potassium ferricyanide in 100 ml of water was added at room temperature.

The reaction mixture was heated up to its boiling point and the reaction was continued at this temperature for two hours.

Filtration, washing, concentration and precipitation as in Example 1.

The purification of the raw compound was carried out in a column filled with activated alumina and eluting with 1,2-ethylenedichloride/n-hexane (50/50 v/v) and subsequently with pure 1,2-ethyleneglycole-dichloride.

The 5-fluoro-2-(5'-nitro-2'-furyl)-benzimidazole thus obtained (yield 51% calculated on 4-fluoro-o-phenylendiamine) had the following characteristics:

-appearance : yellow crystalline solid

-melting point : 225-226°C

-elementary analysis (calculated for $C_{11}H_6O_3N_3F$):

| | | | |
|---|---|---|---|
| found | C=53.30% | H=2.49% | N=17.05% |
| calculated | C=53.44% | H=2.43% | N=17.00% |

Example 4: Preparation of 5-nitro-2-(5'-nitro-2'-furyl)-benzimidazole.

The procedure of Example 1 was followed, preparing a solution of

| | | |
|---|---|---|
| 5-nitro-2-furaldehyde | 15.3 | g |
| 4-nitro-o-phenylendiamine | 13.9 | g |
| methanol | 200.0 | ml |

To this solution, a solution of 66.0 g of potassium ferricyanide in 100 ml of water was added at room temperature.

The reaction mixture was heated up to its boiling point and the reaction was continued at this temperature for two hours.

Filtration as in Example 1. The residue was repeatedly washed with warm acetic acid.Concentration and precipitation as in Example 1.

The purification was carried out in a column filled with activated silica, eluting with chloroform and then via fractional crystallization from an acetic acid/water (90/10 v/v) mixture.

The 5-nitro-2-(5'-nitro-2'-furyl)-benzimidazole thus obtained (yield 64% calculated on 4-nitro-o-phenylendiamine) had the following characteristics:

-appearance : yellow-green crystalline solid

-melting point : >310°C

-elementary analysis (calculated for $C_{11}H_6O_5N_4$):

| | | | |
|---|---|---|---|
| found | C=48.65% | H=2.09% | N=20.15% |
| calculated | C=48.17% | H=2.19% | N=20.44% |

Example 5: Preparation of 5-trifluoromethyl-2-(5'-nitro-2'-furyl)-benzimidazole.

The procedure of Example 1 was followed, preparing a solution of

| | | |
|---|---|---|
| 5-nitro-2-furaldehyde | 15.3 | g |
| 4-trifluoro-methyl-o-phenylendiamine | 16.1 | g |
| methanol | 200.0 | ml |

To this solution, a solution of 66.0 g of potassium ferricyanide in 100 ml of water was added at room temperature.

The reaction mixture was heated up to its boiling point and the reaction was continued at this temperature for three hours.

Filtration, washing, concentration and precipitation as in Example 1.

The purification was carried out via fractional crystallization from an acetone/water (80/20 v/v)mixture.

The 5-trifluoromethyl-2-(5'-nitro-2'-furyl)-benzimidazole thus obtained (yield 69% calculated on 4-trifluoromethyl-o-phenylendiamine) had the following characteristics:

-appearance : pale yellow crystalline solid

-melting point : 230-231°C

-elementary analysis (calculated for $C_{12}H_6O_3N_3F_3$):

| | | | |
|---|---|---|---|
| found: | C=48.65% | H=2.12% | N=14.03% |
| calculated | C=48.48% | H=2.02% | N=14.14% |

Example 6: Preparation of 5-chloro-2-(5'-nitro-2'-furyl)-benzimidazole.

The procedure of Example 1 was followed, preparing a solution of

| | | |
|---|---|---|
| 5-nitro-2-furaldehyde | 15.3 | g |
| 4-chloro-o-phenylendiamine | 13.3 | g |
| methanol | 200.0 | ml |

To this solution, a solution of 66 g of potassium ferricyanide in 100 ml of water was added at room temperature.

The reaction mixture was heated up to its boiling point and the reaction was continued at this temperature for six hours.

Filtration, washing, concentration, precipitation and purification as in Example 1.

The 5-chloro-2-(5'-nitro-2'-furyl)-benzimidazole thus obtained (yield 81% calculated on 4-chloro-o-phenylendiamine) had the following characteristics:

-appearance : yellow-green crystalline solid

-melting point : 234-235°C

-elementary analysis (calculated for $C_{11}H_6O_3N_3Cl$):

| | | | |
|---|---|---|---|
| found | C=50.21% | H=2.30% | N=15.92% |
| calculated | C=50.09% | H=2.27% | N=15.94% |

The nitrofurylbenzimidazoles (I) are particularly active as antimycotic, antibacterial and antitubercular agents.

In fact, they have been found active against various pathogenic microorganisms, such as several strains of Candida, Aspergillus, Cryptococcus and others both in in vitro tests wherein the activity was evaluated via the growth inhibition zone technique on cultures grown on suitable nutrient media and in vivo tests in experiment animals.

The compounds (I) were also shown to be active in vitro and in vivo on Gram+ and Gram- strains.

The antitubercular activity was evaluated in in vitro tests against Mycobacterium aurum.

The evaluation of the growth inhibition zones was carried out with the agar diffusion test disclosed by Elmer W. Koneman et al. Atlas and textbook of diagnostic microbiology - Ed.J.B. Lippincott & Co. (1983) on several strains of bacteria and fungi and on Mycobacterium aurum.

The test results of the compounds of Examples 1 to 6 is illustrated in the following table (values in millimeters).

The nitrofurylbenzimidazoles (I) prepared via the process of the present invention are suitable for being compounded into topically administrable ointments and creams. Moreover because of their low toxicity (for instance $LD_{50}$ in CD1 mice for the compound of Example 3 is 240 mg/kg i.v. ), the compounds (I) are particularly suited for preparing pharmaceutical compositions administrable via the systemic route. Examples of typical formulations (suited for both topical application and systemic administration) are illustrated in the above-mentioned UK patents 1,072,735 and 1,094,903.

Table: Growth inhibition zones (mm) obtained in the agar diffusion test (**Elmer W. Koneman et al: Atlas and textbook of diagnostic microbiology, 1983) with the nitrofurylbenzimidazoles of** formula:

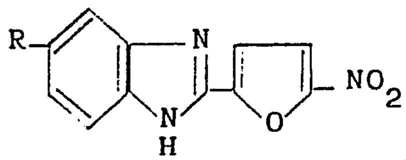

| | Es. 1 | Es. 2 | Es. 3 | Es. 4 | Es. 5 | Es. 6 |
|---|---|---|---|---|---|---|
| | R = H | R = $CH_3$ | R = F | R = $NO_2$ | R = $CF_3$ | R = Cl |
| Staphylocccus aureus ATCC 9144 | 35 | 30 | 28 | 30 | 30 | 28 |
| Staphylococcus epidermis ATCC 14990 | 35 | 32 | 29 | 32 | 30 | 30 |
| Sarcina lutea ATCC 9341 | 15 | 12 | 27 | 12 | 14 | -- |
| Pseudomas aeruginosa ATCC 9321 | -- | -- | -- | -- | -- | -- |
| Escherichia coli ATCC 10538 | 22 | 13 | 21 | 16 | 11 | 15 |
| Streptococcus faecalis ATCC 8043 | 24 | 23 | 33 | 20 | 21 | 22 |
| Salmonella tiphymurium ATCC 13311 | 20 | 12 | 23 | 15 | 13 | 14 |
| Bacillus subtilis ATCC 6051 | 36 | 30 | 27 | 30 | 26 | 28 |
| Enterobacter aerogenes ATCC 13047 | 18 | 16 | -- | 14 | -- | 12 |
| Candida albicans FCA6 | 40 | 32 | 34 | -- | 23 | 22 |
| Candida albicans 1 | 25 | 18 | 34 | -- | -- | 15 |
| Candida albicans 2 | 42 | 33 | 34 | -- | 22 | 22 |
| Candida parapsilosis | 37 | 29 | 34 | -- | 20 | 21 |
| Candida tropicalis | -- | -- | 25 | -- | -- | -- |
| Cryptococcus albidus | 33 | 25 | 35 | -- | -- | -- |
| Cryptococcus neoformans | -- | -- | 45 | -- | -- | -- |
| Rodutorula rubra | -- | -- | 35 | -- | -- | -- |
| Aspergillus fumicatus | 22 | 13 | 30 | -- | -- | -- |
| Mycobacterium aurum NCTC 10437 | 24 | 13 | 35 | 15 | 22 | 13 |
| Torulopsis Candida | -- | -- | 42 | -- | -- | -- |
| Aspergillus Niger | -- | -- | 38 | -- | -- | -- |
| Aspergillus Flavus | -- | -- | 30 | -- | -- | -- |

0252028

Some creamy ointments were prepared by incorporating the benzimidazoles of examples 1 to 6 into the following excipients: rice starch, vaseline oil, solid paraffin, polyethyleneglycol (P.E.G.) 400 and purified water.

The following compositions were prepared (the percentages are by weight)

| Compound of Example | 1 | 2 | 3 | 4 | 5 | 6 |
|---|---|---|---|---|---|---|
| Active principle | 1% | 1% | 1% | 1% | 1% | 1% |
| Starch | 42% | 42% | 42% | 42% | 42% | 42% |
| Vaseline | 7% | 7% | 7% | 7% | 7% | 7% |
| Paraffin | 4% | 4% | 4% | 4% | 4% | 4% |
| P.E.G. 400 | 21% | 21% | 21% | 21% | 21% | 21% |
| Water | 25% | 25% | 25% | 25% | 25% | 25% |

The compositions were prepared as follows:

Solution A: To a polyethyleneglycol solution of benzimidazole, water and starch were added and the resulting solution was stirred thoroughly.

Solution B: The solid paraffin and the vaselin oil were homogenized on a water-bath.

The ointment was obtained by incorporating the solution A into B under heating and thorough stirring. The stirring was continued during the cooling of the ointment.

The ointments thus prepared were used for the topical treatment of some experimental cases of Tinea Corporis caused by M. canis and E.floccosum. After twenty days of treatment the total remission of symptoms was observed and the culture test was negative.

Polyethylene glycol (particularly polyethylene glycol 400) is the excipient of choice for preparing systemically administrable compositions.

What is claimed is:

1. A process for preparing nitrofurylbenzimidazoles having general formula (I)

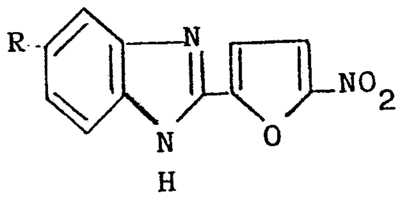

(I)

wherein R is hydrogen;
straight or branched $C_1$-$C_{12}$ alkyl;
halogen;
nitro group; or
halogen-substituted $C_1$-$C_4$ lower alkyl

which comprises condensing an o-phenylendiamine having general formula (II)

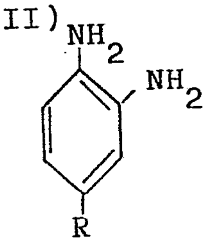

(II)

wherein R has the above-identified meaning, with 5-nitro-2--furaldehyde in the presence of an oxidizing agent, characterized in that the oxidazing agent is selected from the ferricyanides of the alkaline and alkaline-earth metals.

2. The process of claim 1, wherein the ferricyanide is selected from sodium, potassium, rubidium, cesium, calcium, stronzium, barium and magnesium ferricyanide.

3. A systemically administrable pharmaceutical composition having antimycotic activity comprising as antimycotic agent a nitrofurylbenzimidazole having general formula (I)

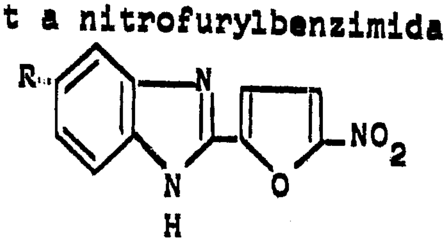

(I)

wherein R is hydrogen;

straight or branched $C_1$-$C_{12}$ alkyl;

halogen;

nitro group; or

halogen-substituted $C_1$-$C_4$ lower alkyl,

and a pharmacologically acceptable excipient therefor.

4. The pharmaceutical composition of claim 3, wherein the excipient is polyethyleneglycol 400.

European Patent Office

# EUROPEAN SEARCH REPORT

0252028

Application number

EP 87 83 0236

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | IL FARMACO, vol. 37, no. 9, 1982, pages 597-611, Pavia, IT; G.A. BISTOCCHI et al.: "Nouveaux dérivés hétérocycliques du benzimidazole à activité germicide"<br>* Page 599 * |  | C 07 D 405/04<br>A 61 K 31/415<br>A 61 K 31/345 |
| D,A | GB-A-1 094 903 (SMITH KLINE & FRENCH LABORATORIES) |  |  |

TECHNICAL FIELDS SEARCHED (Int. Cl.4)

C 07 D 405/00
A 61 K 31/00

The present search report has been drawn up for all claims

| Place of search | Date of completion of the search | Examiner |
|---|---|---|
| THE HAGUE | 02-10-1987 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document